# EUROPÄISCHE PATENTSCHRIFT

(11) **EP 3 067 000 B1**
(45) Veröffentlichungstag und Bekanntmachung des Hinweises auf die Patenterteilung: **13.07.2022**
(21) Anmeldenummer: 16159901.4
(22) Anmeldetag: 11.03.2016
(51) Int. Cl.: A61B 17/34, A61M 25/00, A61M 25/01, A61M 29/00, A61B 17/00

(54) **KANÜLENANORDNUNG**
CANNULA ASSEMBLY
SYSTEME DE CANULE

(30) Priorität: 11.03.2015 AT 501972015; 06.11.2015 AT 509442015
(43) Veröffentlichungstag der Anmeldung: 14.09.2016
(73) Patentinhaber: Klepetko, Walter, 1180 Wien (AT)
(72) Erfinder: Klepetko, Walter, 1180 Wien (AT)
(74) Vertreter: Graf von Stosch, Andreas

(56) Entgegenhaltungen:
- EP-A1- 2 233 169
- WO-A1-92/06733
- WO-A1-2014/071161
- US-A- 5 314 418
- US-A- 6 129 713
- US-A1- 2009 171 276
- US-A1- 2011 160 517
- US-A1- 2013 131 591
- US-A1- 2013 317 438

## Beschreibung

Die Erfindung betrifft eine Kanülenanordnung zum Applizieren einer Strömungskanüle zum Ein-, Durch- oder Umleiten von Strömungsmedien, insbesondere von Blut im menschlichen oder tierischen Körper, wobei die Strömungskanüle einen Hauptabschnitt und einen Spitzenabschnitt aufweist.

Kanülenanordnungen und Kathetersysteme sind seit langer Zeit in vielfältiger Form bekannt. So ist es auch bereits bekannt, Kanülen ausgehend von einem Einschnitt in der Leistengegend über die untere Hohlvene bis zum rechten Vorhof des Herzens zu legen. Es ist auch bekannt, über eine derartige Katheteranordnung mit einem Punktionsdraht und dem vorderen Ende des Katheters das Septum des Herzens zu durchstoßen und somit die Kanüle zum linken Vorhof des Herzens zu legen. Die transseptale Kanülierung des linken Vorhofs erweist sich als notwendig oder vorteilhaft zur temporären Behandlung schwerer pulmonaler Hypertension, sowie zur Anwendung bei konventionellen ECMO-Fällen (Extracorporeal membrane oxygenation) mit starken Sättigungsproblemen.

Schwerer Lungenhochdruck im Endstadium kann nur durch eine Transplantation der Lunge behandelt werden. Die Wartezeit auf ein passendes Spendeorgan kann jedoch in extremen Fällen nur durch eine mechanische Entlastung der pulmonalen Zirkulation überbrückt werden. Die derzeit dafür etablierte Methode stellt eine periphere venoarterielle ECMO dar, die jedoch ein beträchtliches Risiko für den Patienten darstellt.

Mit der vorliegenden Kanülenanordnung gelingt es, durch interventionelle transseptale Kanülierung des linken Vorhofs und zusätzlich der Pulmonalarterie einen Umgehungskreislauf für die PH-Strombahn (PH= Pulmonale Hypertonie) herzustellen, wodurch es ermöglicht wird, den Patienten über längere Zeit zu stabilisieren. Bei dem vorgenannten Krankheitsbild kann die Vorgangsweise folgende Schritte umfassen:
- transseptale Kanülierung des linken Vorhofs (arteriell return),
- transtricuspidale Kanülierung der Pulmonalarterie (venus drainage) oder
- normale venöse Kanülierung,
- Konnektierung der Kanülen mit einer herkömmlichen Novalung ^{®} (bei transtricuspidaler Kanülierung) oder mit ILA-active (bei venöser Kanülierung) oder mit vergleichbaren Geräten.

Aus der europäischen Patentanmeldung EP 2233169 A1 ist eine transseptale Kanüle mit einem flexiblen Kanülenkörper bekannt, die mit einem linken atrialen Verankerungselement am distalen Ende des flexiblen Kanülenkörpers und einem rechten atrialen Verankerungselement betriebsfähig befestigt werden kann. Die linken und rechten Vorhofanker können dabei implantiert und separat eingesetzt werden. Die transseptale Kanüle weist ferner einen koaxialen Ballonkatheter und einen Rohrkörper mit einem inneren Element und einem äußeren Element auf, welches rund um das innere Element angeordnet ist, wodurch ein Aufblaskanal zwischen dem inneren und dem äußeren Element geschafft wird. Ferner weist die transseptale Kanüle eine Nabe und einen Fluidraum auf, der in Fluidverbindung mit dem Aufblaskanal ist, wobei ein Ballon des Ballonkatheters in Fluidverbindung mit dem Aufblaskanal ist. Die Nabe des koaxialen Ballonkatheters ist dabei mit einem niedrigen Profil derart ausgebildet, dass andere chirurgische Vorrichtungen über die koaxiale Nabe ohne Entleeren und Entfernen des Ballons geführt werden können.

Aus der US Patentanmeldung US 2010 160517 A1 ist eine Kanüle bekannt, die einen länglichen Körper mit einer Länge von mindestens 70 cm und die einen Kanal aufweist, der eine Wand definiert. Die Kanüle beinhaltet einen Draht, der zumindest innerhalb eines Teils der Wand angeordnet ist. Der Kanülenkörper hat ein proximales Ende und ein distales Ende, das eine Spitzenöffnung aufweist, durch welche der Kanal hindurchführt. Der Kanülenkörper weist eine Vielzahl von Seitenlöchern durch die Wand in der Nähe der Spitze an dem distalen Ende auf. Ferner enthält die Kanüle eine Schlauchtülle an ihrem proximalen Ende. Die Kanüle beinhaltet ferner einen Nahtflügel, um den länglichen Kanülenkörper festlegen zu können.

Aus der internationalen Patentanmeldung WO 92/06733 A1 ist ein System für die Kannülierung des linken Vorhofs durch das Septum bekannt, durch das eine Drainage von Blut im linken Vorhof möglich ist. Dabei werden ein Führungsdraht und eine lange Nadelanordnung in einen Katheter eingeführt. Eine Kanüle wird über die Außenseite des Katheters geführt. Der Führungsdraht kann an der Nadelanordnung vorbeigeschoben werden und durch den Katheter hindurch durch dessen distales Ende um das Einbringen des Systems in das rechte Atrium zu assistieren. Die Nadel ist dabei dazu ausgelegt, das Septum zu penetrieren, so dass der Katheter über die Nadel hinwegbewegt werden kann, um die septale Öffnung zu erweitern.

Aus der US Patentanmeldung US 2013/0317438 A1 ist ein System für die Erstellung eines transluminalen Gefäßzugangs bekannt, das aus einem Dilatoradapterelement mit einem proximalen und einem distalen Ende und einem in diesem befindlichen Dilatoradapterlumen besteht. Das Dilatoradapterlumen ist dabei durch ein Innendurchmesserprofil definiert und so bemessen, einen oder mehrere Abschnitte eines Führungsdrahts aufzunehmen. Der Dilatoradapter ist weiterhin definiert durch einen Außendurchmesser, der so bemessen ist, dass der Dilatoradapter wenigstens teilweise zu einem proximalen Ende in ein Dilatorteillumen einführbar ist, welches durch ein Dilatorelement gebildet ist. Das Dilatorelement ist dabei an ein Einführungskatheterelement koppelbar durch ein Einführungselementlumen, welches in dem Einführungselement ausgebildet ist.

Aus dem US Patent US 5314418 ist eine mit dem linken Vorhof verbundene Entleerungskanüle bekannt. Die Entleerungskanüle umfasst einen Spitzenabschnitt und einen Krümmungsabschnitt, der mit dem Spitzenabschnitt verbunden ist. Die Entleerungskanüle weist einen rohrfömigen Abschnitt auf, der mit dem Krümmungsabschnitt verbunden ist. Die Länge und der Außendurchmesser der Kanüle ist ausreichend, um sie in die Oberschenkelvene einzuführen und den Spitzenabschnitt durch die Vena cava im linken Vorhof in den rechten Vorhof zu gelangen und an dem interatrialen Septum vorbeizugelangen. Der Krümmungsabschnitt ist dabei aus einem Material hergestellt, das flexibler ist als der röhrförmige Abschnitt. Eine elastische Drahtspirale ist innerhalb der Wand des Wandteils angeordnet, die das Lumen des Krümmungsabschnitts definiert.

Immer dann, wenn Kanülen Gewebeabschnitte durchstoßen müssen, wie dies z.B.: beim Septum für die Kanülierung des linken Vorhofs notwendig ist, besteht das Problem, dass die Kanüle den für das Durchstoßen notwendigen Punktionsdraht mit dessen Punktionsspitze möglichst unter einem 90-grädigen Winkel auf den Gewebeabschnitt richten soll. Dies ist beim Septum des Herzens ein geringeres Problem, wenn die Kanülierung femoral erfolgt, also über die untere Hauptvene, da dann die in den linken Vorhof geführte Kanüle und der in ihr geführte Punktionsdraht annähernd mit 90°-igem Winkel an das Septum zu liegen kommt. Bei den derzeit bekannten Kanülenanordnungen ist es aber nicht möglich, den bevorzugten Zugang von der oberen Körperhälfte zu schaffen. Der Zugang von oben, beispielsweise über die obere Hohlvene, cervical oder subklavikulär, ist für den Patienten wesentlich angenehmer. Der Zugang hat eine bessere Dauerhaftigkeit und geringere Problemanfälligkeit. Weiters soll die Kanülenanordnung eine Verankerung im Septum zur Verhinderung von Dislokation aufweisen können. Die letztendlich erzielte Durchflussmenge muss ausreichend groß sein. Überdies ist es wünschenswert, die Kanüle und auch die sonstigen Teile der Kanülenanordnung mit antithrombotischen Eigenschaften zu versehen. Bevorzugt soll die Anordnung auch die Möglichkeit des Defektverschlusses im atrialen Septum bei eventueller Entfernung bieten. Und letztendlich soll die Kanülenanordnung auch die Konnektierung mit dem Novalung ^{®} - System in einer Weise ermöglichen, die dauerhaft und stabil ist, einen leichten und raschen Wechsel der Novalung ^{®} ermöglicht und den Patienten in physischen Aktivitäten möglichst wenig behindert.

US 6129713 A offenbart eine Kanülenanordnung mit einer Kanüle die eine vorgeformt gewinkelte Konfiguration des distalen Endes aufweist.

Erfindungsgemäß wird die generelle Aufgabe der Kanülierung mit annähernd 90°-igem Punktieren eines Gewebeabschnittes und insbesondere das Setzen einer Kanüle durch das Septum des Herzens mit einer Kanülenanordnung gemäß Anspruch 1 dadurch gelöst, dass der Spitzenabschnitt elastisch und in Funktionslage bogenförmig in einem Winkel vom Hauptabschnitt abstehend ausgebildet ist, und dass der Spitzenabschnitt in Applikationsanlage durch eine Führungsvorrichtung in Richtung der Längserstreckung des Hauptabschnittes gehalten ist und dass die Kanülenanordnung zur transseptalen Kanülierung des linken Vorhofes des Herzens mit Zugang über die obere Hohlvene zur transatrialen Punktion geeignet ist.

Weitere Merkmale der Erfindung sind unter anderem die folgenden:
Die Führungsvorrichtung weist bevorzugt einen Führungsdraht, eine über den Führungsdraht schiebbare erste Dilatationskanüle, eine über die erste Dilatationskanüle schiebbare zweite Dilatationskanüle und eine über der zweiten Dilatationskanüle angeordnete Führungshülse auf.

Ein weiteres bevorzugtes Merkmal besteht darin, dass die erste und zweite Dilatationskanüle in ihren Spitzenabschnitten in gleicher Weise wie der Spitzenabschnitt der Kanüle elastisch bogenförmig von den jeweiligen Hauptabschnitten in einem Winkel abstehend ausgebildet sind, wobei die erste Dilatationskanüle durch den Führungsdraht und die zweite Dilatationskanüle durch die in ihr angeordnete erste Dilatationskanüle geführt und entlang der Längserstreckung des Führungsdrahtes geführt ist.

Weiters ist es vorteilhaft, wenn die Führungshülse bei der Applikation vom Spitzenabschnitt entfernbar ist, sodass dieser die bogenförmige Krümmung einnehmen kann, und die Führungshülse zur Gänze entfernbar und durch die Kanüle ersetzbar ist, wonach der Führungsdraht und die ersten und zweiten Dilatationskanülen aus der Kanüle entfernbar sind, sodass der gesamte Innenquerschnitt der Kanüle freigegeben ist.

Weiters kann die Erfindung dadurch gekennzeichnet sein, dass der Spitzenabschnitt der zweiten Dilatationskanüle benachbart zur Spitze einen Dilatationsballon aufweist, dessen Durchmesser im aufgeblasenen Zustand dem Außendurchmesser der Kanüle entspricht und dessen Durchmesser im entspannten Zustand gleich oder kleiner als der Innendurchmesser der Kanüle ist.

Die Kanüle kann im Spitzenabschnitt eine die Kanüle fixierende Arretiervorrichtung, bevorzugt einen Ballon, aufweisen.

Die erste und zweite Dilatationskanüle und die Kanüle ist bevorzugt im Spitzenabschnitt in ungestütztem Zustand um einen Winkel von 20° bis 100°, bevorzugt 60° bis 95°, besonders bevorzugt 90°, gebogen.

Die Oberflächen der Teile der Anordnung sind bevorzugt antithrombotisch ausgerüstet, insbesondere heparinisiert.

Nachfolgend wird die Erfindung anhand der Zeichnungen beispielsweise näher erläutert.

Fig. 1 zeigt schematisch eine Kanülenanordnung gemäß Stand der Technik.

Fig. 2 bis 7 zeigen schematisch schrittweise den Aufbau und die Anwendung der erfindungsgemäßen Anordnung beim menschlichen Herzen für das Setzen der Strömungskanüle in den linken Vorhof des Herzens.

Fig. 8 zeigt eine andere schematische Herzdarstellung zur Erklärung des Weges eines zusätzlichen Pulmonaliskatheters und Fig. 9 die Anordnung einer Vorrichtung zur extrakorporalen Membranoxidation.

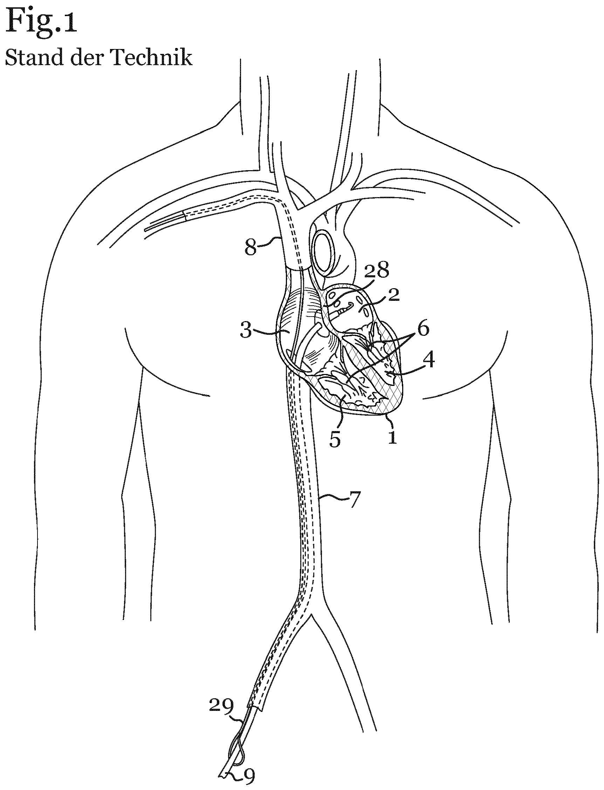

Die Fig. 1 zeigt den Stand der Technik, wie er beispielsweise durch die US 8 343 029 B2 geoffenbart wurde. Ein Katheter 28 wird bevorzugt durch einen Einschnitt in der Leistengegend durch die untere Hohlvene 7 nach oben in den rechten Vorhof 3 des Herzens 1 geschoben und durchdringt mit seinem Spitzenabschnitt das Septum 28, sodass der Katheter im linken Vorhof 2 endet. Diese Lage des Katheters und der damit eingebrachten Kanüle, die den Körper von der Leistengegend bis zum Herz durchragt, weist jedoch die eingangs beschriebenen Nachteile auf.

Die Fig. 1 zeigt als Stand der Technik auch eine eingelegte Schlaufe 29, die von der Vene im Bereich des Schlüsselbeins über die obere Hohlvene 8 nach unten geführt werden kann, um einen Führungsdraht, der in dem Katheter 9 vorgesehen ist, nach oben zu ziehen. Damit ist es aber nicht möglich eine Kanüle mit entsprechendem Volumen und einem Durchmesser von etwa 5 bis 9 mm bis in die linke Vorhofkammer zu applizieren.

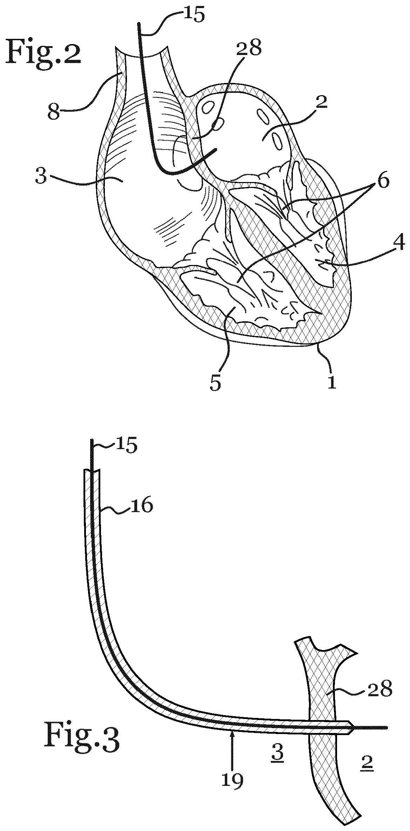

Der Vorgang unter Anwendung der erfindungsgemäßen Kanülenanordnung wird anhand der Figuren 2 bis 7 erläutert.

Als erster Schritt wird gemäß Fig. 1 in bekannter Weise mittels des Katheters 9 der Führungsdraht 15 mit seiner Punktionsspitze durch das Septum 28 hindurch in den linken Vorhof 2 gebracht. Sodann wird der Führungsdraht - nach Entfernung des Katheters 9 - mit der Schlaufe 29 nach oben in die Halsvene oder die Schlüsselbeinvene gezogen, wobei das Ende des Führungsdrahtes durch eine Öffnung der Vene aus dem Körper herausgeführt ist.

In Fig. 2 ist gezeigt, wie der Führungsdraht 15 von der oberen Hohlvene 8 bis zum linken Vorhof 2 geführt ist. Dieser Führungsdraht - wobei das Wort "Draht" keine Einschränkung auf das Material darstellt - kann sehr weich und schmiegsam sein. Falls sich herausstellt, dass der Führungsdraht 15 zu weich ist, um die erste Dilatationskanüle 16 angemessen zu führen, kann der Führungsdraht dadurch gegen einen steiferen Führungsdraht ausgetauscht werden, indem ein weiches Rohr, auch sheet genannt, über den weichen Draht bis in das Septum geschoben, der weiche Draht entfernt und der steife Draht als neuer Führungsdraht 24 bis zum linken Vorhof eingeschoben wird.

Ein steifer Draht kann aber auch später eingezogen werden, wenn die folgenden Dilatationskanülen schon gesetzt sind.

Gemäß Fig. 3 wird als nächster Schritt die erste Dilatationskanüle 16 über den Führungsdraht 15 oder 24 eingeschoben und die erste Dilatationskanüle 16 weist einen Spitzenabschnitt 19 auf, der mit ausreichender Spannung vorgebogen ist (jedoch nur so stark, dass er noch vom Führungsdraht gestreckt werden kann), sodass die erste Dilatationskanüle in etwa 90°-igem Winkel gegen das Septum 28 zu liegen kommt und durch das Septum 28 durchgestoßen werden kann. Damit ist ein erster noch relativ weicher und enger Zugang von oben zur linken Vorhofkammer des Herzens geschaffen.

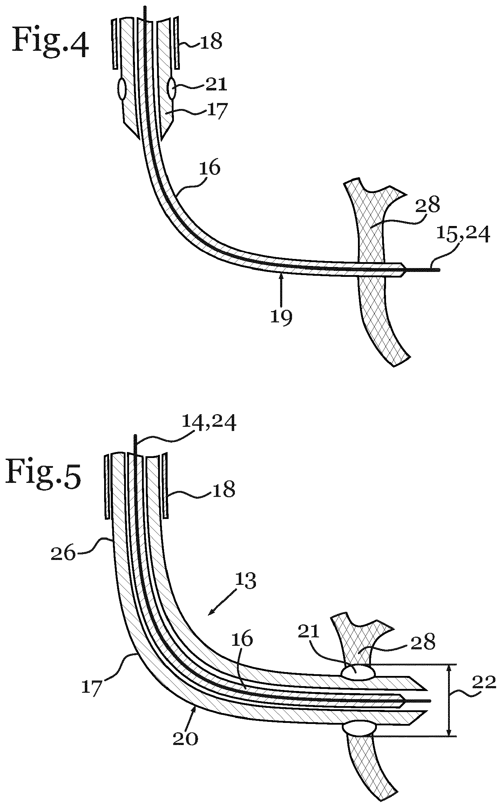

Gemäß Fig. 4 wird im nächsten Schritt über und entlang der ersten Dilatationskanüle 16 eine zweite Dilatationskanüle 17 geschoben. Auch die zweite Dilatationskanüle 17 ist im Spitzenabschnitt 20 mit genügender Vorspannung um etwa 90° gegenüber deren Hauptabschnitt 26 vorgebogen. Mit Hilfe der außen angeordneten Führungshülse 18 wird jedoch der Spitzenabschnitt 20 der zweiten Dilatationskanüle derart gerade gehalten, dass die Kanüle über den gesamten Hauptabschnitt der ersten Dilatationskanüle 16 gerade vorgeschoben werden kann. Zur Versteifung der zweiten Dilatationskanüle trägt auch die erste Dilatationskanüle ihren Beitrag bei. Sobald die zweite Dilatationskanüle in die Stellung gemäß Fig. 4 kommt, wird die Führungshülse 18 vom Arzt daran gehindert, die Vorschubbewegung der zweiten Dilatationskanüle mitzumachen, sodass der Spitzenabschnitt die Führungshülse 18 verlässt und entsprechend der Vorbiegung die gebogene Stellung einnimmt, wie in Fig. 5 dargestellt ist. Die ungestützte Lage der Kanülen gemäß Fig. 5 wird hier als Funktionslage bezeichnet wohingegen die gestützte, gestreckte Lage der jeweiligen Spitzenabschnitte als Applikationslage bezeichnet wird.

Wie in Fig. 5 ebenfalls dargestellt ist, wird auch die zweite Dilatationskanüle 17 bis in den linken Vorhof 2 durch das Septum 28 geschoben, wobei im Spitzenabschnitt der zweiten Dilatationskanüle bevorzugt ein Dilatationsballon 21 vorgesehen ist, der in bekannter Weise aufgeblasen werden kann, um das Loch im Septum 28 für die endgültig einzuführende Kanüle zu erweitern.

Als nächster Schritt wird die Führungshülse 18 von den Dilatationskanülen nach außen abgezogen und es wird die letztendlich benötigte Kanüle 10 eingeschoben, die ebenfalls einen vorgebogenen Spitzenabschnitt aufweisen kann, wobei während der Applikation beim Einschieben über die beiden vorher eingebrachten Dilatationskanülen die notwendige Steifheit gegeben ist, sodass die Vorbiegung überwunden wird und die Kanüle 10 in gerader Form eingeschoben werden kann.

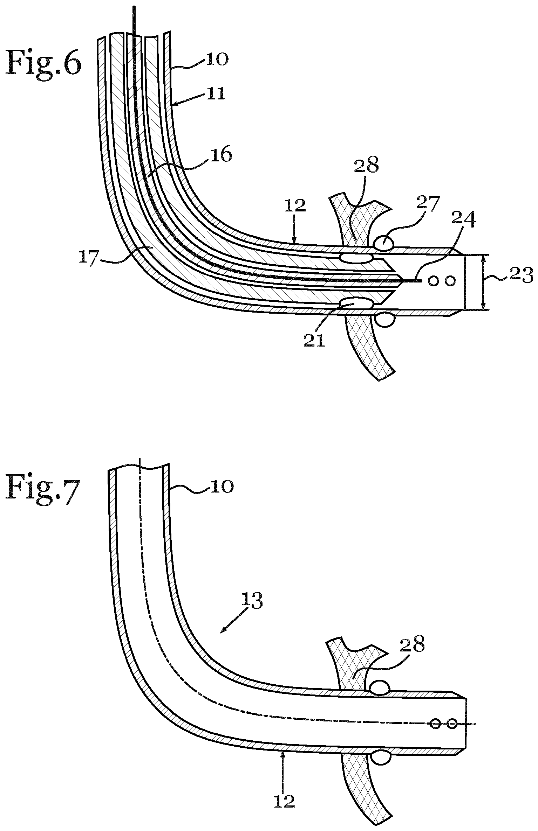

Sobald die Biegung gemäß Fig. 6 erreicht ist, kann die Kanüle 10 der Biegung leicht folgen und durch das Septum hindurch in den linken Vorhof eingeschoben werden.

Zur Arretierung der Kanüle ist ein Arretierballon eingezeichnet, der als Arretiervorrichtung 27 ballonförmig ist. Die Arretierung kann aber auch durch andere Mittel erfolgen, wie z.B. Verspreitzungen.

Wie in Fig. 6 ebenfalls zu sehen ist, kann der Dilatationsballon 21 der zweiten Dilatationskanüle 17 durch Auslassen des Luftdrucks derart entspannt werden, dass der Außendurchmesser im Ballonbereich etwa dem Innendurchmesser der Kanüle 10 entspricht. Damit wird der Kanüle 10 der Weg durch das Septum freigegeben. Im aufgeblasenem Zustand gemäß Fig. 5 weist der Ballon 21 einen Außendurchmesser auf, der dem Außendurchmesser der Kanüle 10 entspricht und somit das Loch im Septum derart ausweitet, dass die Kanüle 10 leicht eingeschoben werden kann.

Die Fig. 7 zeigt letztendlich die fertige Stellung der Kanüle 10, aus der alle Innenteile nämlich Führungsdraht 15 oder 24 und beide Dilatationskanülen 16, 17 herausgezogen worden sind. Somit steht das volle Querschnittsvolumen der Strömungskanüle 10 als Linke-Vorhof-Kanüle (LA-Kanüle) zur Verfügung, um die gewünschten Durchflussmengen und Strömungsbedingungen herzustellen.

Die Zahl der übereinander zu schiebenden Dilatationskanülen ist nicht auf zwei beschränkt. Wenn der Dilatationsballon 21 vermieden werden soll, kann auch noch eine dritte oder vierte Kanüle mit den oben genannten Eigenschaften übergezogen werden, um die notwendige Erweiterung des Loches im Septum und den erforderlichen Strömungsquerschnitt in der letzten Endes eingesetzten Strömungskanüle 10 zu erreichen. Die Dilatationskanülen weisen bevorzugt einen inneren freien Querschnitt auf, der das Schieben der Kanüle über den innenliegenden Führungsdraht oder über die innenliegende Dilatationskanüle erlaubt.

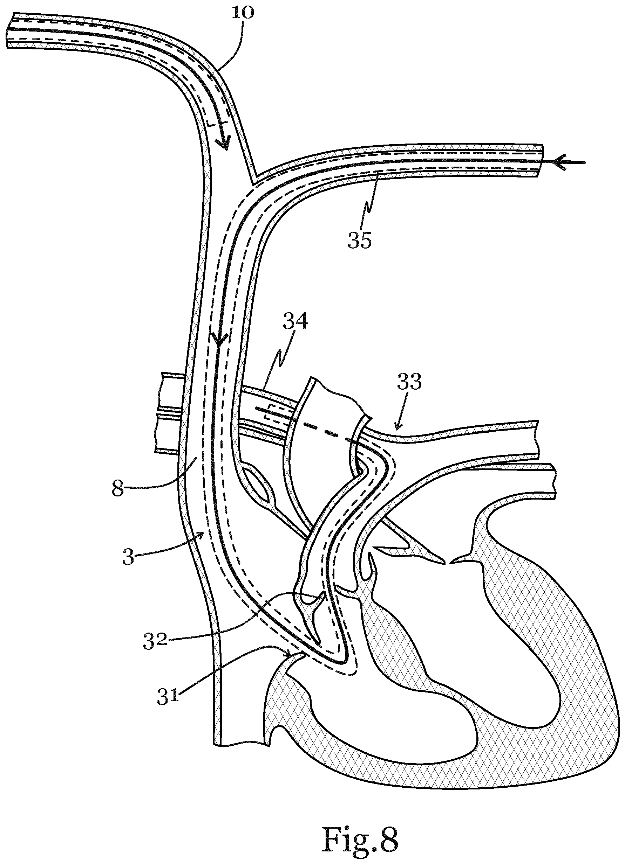

Die Fig. 8 ist eine schematische Darstellung der Anordnung einer weiteren Strömungskanüle als Pulmonaliskanüle 35 für die Kanülierung der Pulmonalisaterie 33. Dabei wird über die obere Hohlvene 8 ein herkömmlicher Pulmonaliskatheter eingesetzt, der relativ weich ist und einen Ballon trägt. Ein solcher Pulmonaliskatheter kann mit einem Führungsdraht ausgestattet werden, sodass der Führungsdraht mit dem Katheter durch den natürlichen Blutstrom über den rechten Vorhof 3, durch die Trikuspidalklappe 31 in die rechte Herzkammer und von dort durch die Pulmonalklappe 32 in die Pulmonalarterie 33 geschwemmt wird. Bevorzugt soll der Führungsdraht in die rechte Pulmonalarterie 34 eingesetzt werden.

Sobald der Führungsdraht seine gewünschte Lage angenommen hat, können in der bisher beschriebenen Weise schrittweise aufeinanderfolgend eine oder mehrere Kanülen eingeschoben werden, wobei durch den Führungsdraht die Führung übernommen wird und die Kanülen durch entsprechende Flexibilität die Biegung in die gewünschte Form mitmachen können.

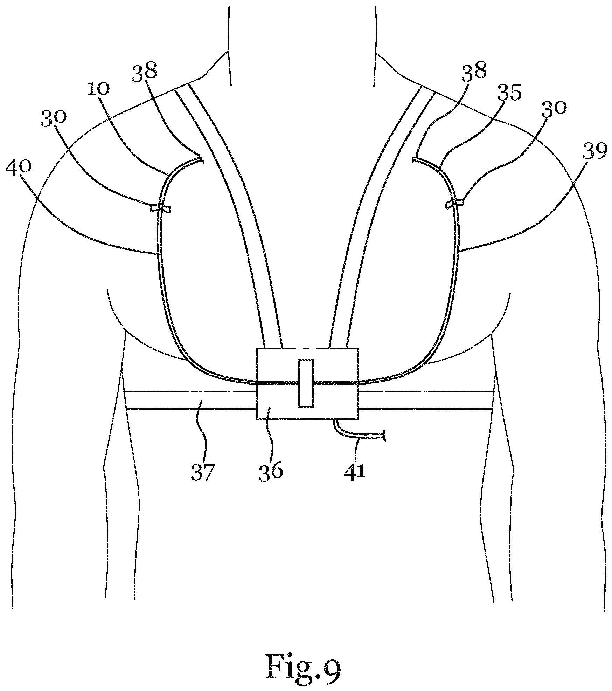

Mithilfe der gemäß Fig. 2 bis 7 in den linken Vorhof eingeführten ersten Strömungskanüle 10 (LA-Kanüle) und der gemäß Fig. 8 in die Pulmonalarterie eingeführten Pulmonaliskanüle 35 ist es möglich, Blut aus der Pulmonalarterie in den linken Vorhof abzuleiten, wobei über entsprechende Schlauchverbindungen außerhalb des Körpers eine tragbare Vorrichtung angeschlossen werden kann, in der aus dem Blut CO2 entfernt und 02 zugeführt wird. Dies ist in Fig. 9 dargestellt.

In Fig. 8 ist die zur linken Vorhofkammer führende Strömungskanüle 10 (LA-Kanüle) nur im oberen Abschnitt angedeutet. Die vollständige Lage ist den Fig. 2 bis 7 zu entnehmen.

Alternativ kann anstelle der Blutentnahme aus der Pulmonalarterie eine venöse Blutentnahme erfolgen, beispielsweise aus der oberen Hohlvene. Mithilfe einer Pumpenunterstützung (insbesondere einer Kreiselpumpe) kann das venöse Blut über die Novalung oder über ein vergleichbares Gerät und die LA-Kanüle in den linken Vorhof des Herzens zurückgeführt werden. Diese Alternative ist für jene Fälle bevorzugt, wenn die pulmonale Hypertonie nicht übermäßig stark ausgeprägt ist und die Lungenfunktion ersetzt oder unterstützt werden soll.

Die Vorgangsweise und weitere Ausführungen werden im Folgenden beschrieben:
Lungenhochdruck im Endstadium führt zum rechtsventrikulären Versagen. Wenn die medikamentöse Therapie ausgeschöpft ist, kann dieses Versagen nur durch eine mechanische Kreislaufunterstützung verhindert werden. Alle bisherigen beispielhaften Verfahren sind entweder operativ zu installieren, oder nur für eine kurzzeitige Anwendung ohne Möglichkeit für Mobilität des Patienten geeignet.

Die perkutane Einbringung von Kanülen über einen Zugang von der oberen Körperhälfte ermöglicht es, einen dauerhaften Zugang herzustellen. In Verbindung mit einer Membran, die CO2 entfernt und O2 zuführt (z.B. Novalung, ILA = interventioneller Lungassist) kann man Blut von der Pulmonalarterie in den linken Vorhof umleiten. Dies ermöglicht es, eine Druckreduzierung in der Pulmonalarterie zu erreichen, und gleichzeitig die Sauerstoffsättigung des arteriellen Blutes aufrecht zu erhalten. Der Blutfluss erfolgt dabei rein passiv, getrieben durch die hohe Druckeffizienz zwischen Pulmonalarterie und linken Vorhof, oder wenn dies nicht ausreichend ist, durch einen zusätzlichen Pumpenantrieb, wie zum Beispiel mittels Kreiselpumpe.

Durch die erfindungsgemäße entsprechende Gestaltung der Kanülen in Verbindung mit einer Tragtaschenkonstruktion kann eine eingeschränkte Mobilität der Patienten erzielt werden.

Um dieses Konzept zu verwirklichen bedarf es:
- einer geeigneten Kanüle zum Ableiten des Blutes aus der Pulmonalarterie, nämlich der Pulmonaliskanüle 35,
- einer geeignet Konnektionsmöglichkeit dieser Kanüle mit einem Konnektor 30, wie er bekannt ist,
- einer Novalung-Membran 36 (mit oder ohne Pumpunterstützung) oder eines vergleichbaren Gerätes,
- einer geeignet Konnektionsmöglichkeit 30 der Novalung mit der
- Kanüle zum Rückführen des Blutes in den linken Vorhof, nämlich der LA-Kanüle 10.

Weiters ist die Tragtaschenkonstruktion 37 vorgesehen.

Die Pulmonaliskanüle wird durch perkutane Punktion der Vena Subclavia, bevorzugt der linken Vena Subclavia eingebracht. Folgende Schritte sind dazu notwendig:
- Punktion der Vene an der Cision 38
- Einbringen eines ersten weichen Führungsdrahtes
- Darüber Einbringen eines Katheters mit Ballon an der Spitze. Diesen ist ähnlich wie einem herkömmlichen Pulmonaliskatheter, aber geeignet um als Führung für einen darin einzubringenden steiferen Führungsdraht zu dienen. Dieser Katheter wird durch Aufblasen des Ballons passiv durch die obere Hohlvene, die Trikuspidalklappe 31, die Pulmonalklappe 32 in die Pulmonalarterie durch den Blutfluss eingeschwemmt. Danach wird ein zweiter steifer Führungsdraht innerhalb des Ballonkatheters in die Pulmonalarterie vorgeschoben und der Ballonkatheter entfernt.
- Nunmehr wird die eigentliche Pulmonaliskanüle über den in der Pulmonalarterie liegenden Führungsdraht eingebracht, und dort positioniert. Danach wird der Führungsdraht entfernt. Wenn notwendig kann als Zwischenschritt noch eine Führungskanüle mit erweitertem Querschnitt eingesetzt werden.

Die Pulmonaliskanüle 35 hat an ihrem proximalen Ende eine Konnektionsvorrichtung 30, die eine rasche, einfache und stufenlose Verbindung mit der Novalung ermöglicht. Dies geschieht durch eine geeignete erste Konnektionskanüle 39, sodass die notwendige Konfiguration erreicht wird, um einen direkten und ungehinderten Zugang zur Novalung zu ermöglichen.

Die verwendete Novalung entspricht den herkömmlich verwendeten Membranvorrichtungen. Diese kann in einer geeigneten Tragevorrichtung 37 so vor der Brust des Patienten montiert werden, dass dieser zwar eingeschränkt aber doch mobil ist; siehe dazu Fig. 9. Die notwendige Sauerstoffzufuhr erfolgt durch einen Sauerstoffschlauch 41 aus einem separaten Sauerstoffreservoir (nicht dargestellt). Im Falle der Verwendung einer ILA active mit einem Pumpsystem ist die Anbringung der ganzen Vorrichtung auf einer Tragekonsole möglich. Dementsprechend sind die Konnektoren in einer anderen dafür geeigneten Konfiguration zu gestalten, was jedem Techniker der Medizintechnik geläufig ist.

In ähnlicher Weise ist eine zweite Konnektionskanüle 40 erforderlich, die die notwendige Verbindung zwischen Novalung 36 und Linksvorhofkanüle (LA-Kanüle 10) ermöglicht.

Die Linksvorhofkanüle wird entsprechend den Fig. 2 bis Fig. 7 eingebracht, die dann oberhalb durch die obere Hohlvene 8 in das Herz bis zum linken Vorhof geführt ist.

Diese Anordnung kann für einen temporären Einsatz, zum Beispiel als Überbrückung bis zu einer Transplantation, oder einer Verbesserung des Gesamtzustandes durch allgemeine Erholung des Patienten, eventuell auch nach Operationen verwendet werden. Sie kann aber auch als permanente Unterstützung bei Patienten dienen, die keine Aussicht auf Verbesserung haben und keine Kandidaten für eine Transplantation darstellen.

### Bezugszeichenliste

- 1.: Herz
- 2.: Linker Vorhof
- 3.: Rechter Vorhof
- 4.: Linke Kammer
- 5.: Rechte Kammer
- 6.: Herzklappen
- 7.: Untere Hohlvene
- 8.: Obere Hohlvene
- 9.: Katheter
- 10.: Strömungskanüle (LA-Kanüle)
- 11.: Hauptabschnitt Kanüle
- 12.: Spitzenabschnitt Kanüle
- 13.: Winkel
- 14.: Führungsvorrichtung
- 15.: Führungsdraht weich
- 16.: Erste Dilatationskanüle
- 17.: Zweite Dilatationskanüle
- 18.: Führungshülse
- 19.: Spitzenabschnitt erster Dilatationskanüle
- 20.: Spitzenabschnitt zweite Dilatationskanüle
- 21.: Dilatationsballon
- 22.: Durchmesser Dilatationsballon aufgeblasen
- 23.: Durchmesser Dilatationsballon entspannt
- 24.: Führungsdraht steif
- 25.: Hauptabschnitt erste Dilatationskanüle
- 26.: Hauptabschnitt zweite Dilatationskanüle
- 27.: Arretiervorrichtung
- 28.: Septum
- 29.: Schlaufe
- 30.: Konnektor
- 31.: Trikuspidalklappe
- 32.: Pulmonalklappe
- 33.: Pulmonalarterie
- 34.: Rechte Pulmonalarterie
- 35.: Pulmonaliskanüle
- 36.: Novalung-Membran
- 37.: Tragegestell
- 38.: Cision
- 39.: Konnektionskanüle
- 40.: Konnektionskanüle
- 41.: Sauerstoffschlauch

## Patentansprüche

1. Kanülenanordnung zum Applizieren einer Strömungskanüle zum Ein-, Durch- oder Umleiten von Strömungsmedien, insbesondere Blut, im menschlichen oder tierischen Körper, aufweisend die Strömungskanüle (10) und eine Führungsvorrichtung (14), wobei die Strömungskanüle (10) einen Hauptabschnitt (11) und einen Spitzenabschnitt (12) aufweist, wobei der Spitzenabschnitt (12) vorgebogen ist und elastisch und in Funktionslage bogenförmig in einem Winkel vom Hauptabschnitt (11) abstehend ausgebildet ist und wobei der Spitzenabschnitt (12) in Applikationslage durch die Führungsvorrichtung (14) in Richtung der Längserstreckung des Hauptabschnittes (11) gehalten ist,
**dadurch gekennzeichnet, dass** die Kanülenanordnung zur transseptalen Kanülierung des linken Vorhofes (2) des Herzens (1) mit Zugang über die obere Hohlvene zur transatrialen Punktion ausgebildet ist.

2. Kanülenanordnung nach Anspruch 1, **dadurch gekennzeichnet, dass** die Führungsvorrichtung (14)
- einen Führungsdraht (15, 24, 30),
- eine über den Führungsdraht schiebbare erste Dilatationskanüle (16),
- eine über die erste Dilatationskanüle (10) schiebbare zweite Dilatationskanüle (17) und gegebenenfalls noch weiterer Dilatationskanülen und
- eine über der zweiten oder äußersten Dilatationskanüle angeordnete Führungshülse (18)
umfasst.

3. Kanülenanordnung nach Anspruch 2, **dadurch gekennzeichnet, dass** die erste und zweite und gegebenenfalls jede weitere Dilatationskanüle (16, 17) in ihren Spitzenabschnitten (19, 20) in gleicher Weise wie der Spitzenabschnitt (12) der Strömungskanüle (10) mit ausreichender Spannung vorgebogen sind und elastisch bogenförmig von den jeweiligen Hauptabschnitten (11, 25, 26) in einem Winkel abstehend ausgebildet sind, wobei die erste Dilatationskanüle (16) durch den Führungsdraht (15, 24) und die zweite Dilatationskanüle (17) und gegebenenfalls jede weitere durch die in ihr angeordnete erste Dilatationskanüle (16) geführt und entlang der Längserstreckung des Führungsdrahtes (24) geführt ist.

4. Kanülenanordnung nach einem der Ansprüche 1 bis 3, **dadurch gekennzeichnet, dass** die Führungshülse (18) bei der Applikation vom Spitzenabschnitt (12) entfernbar ist, sodass dieser die bogenförmige Krümmung einnehmen kann, und dass die Führungshülse (18) zur Gänze entfernbar und durch die Strömungskanüle (10) ersetzbar ist, wonach der Führungsdraht (24) und die ersten und zweiten und etwaige weitere Dilatationskanülen (16, 17) auf der Strömungskanüle (10) entfernbar sind, sodass der gesamte Innenquerschnitt der Strömungskanüle (10) freigegeben ist.

5. Kanülenanordnung nach einem der Ansprüche 2 bis 4, **dadurch gekennzeichnet, dass** der Spitzenabschnitt (20) der zweiten oder äußersten Dilatationskanüle (17) benachbart zur Spitze einen Dilatationsballon (21) aufweist, dessen Durchmesser (22) im aufgeblasenem Zustand dem Außendurchmesser der Kanüle entspricht und dessen Durchmesser (23) im entspannten Zustand kleiner als der oder gleich dem Innendurchmesser der Kanüle ist.

6. Kanülenanordnung nach einem der Ansprüche 1 bis 5, **dadurch gekennzeichnet, dass** die Strömungskanüle (10) im Spitzenabschnitt (12) eine die Kanüle (10) fixierende Arretiervorrichtung (27), bevorzugt einen Ballon, aufweist.

7. Kanülenanordnung nach einem der Ansprüche 1 bis 6, **dadurch gekennzeichnet, dass** die erste, zweite und eventuell folgende Dilatationskanülen (16, 17) und die Strömungskanüle (10) im Spitzenabschnitt (12) in ungestütztem Zustand um einen Winkel von 20° bis 100°, bevorzugt 60° bis 95°, besonders bevorzugt 90°, gebogen ist.

8. Kanülenanordnung nach einem der Ansprüche 1 bis 7, **dadurch gekennzeichnet, dass** die Oberflächen der Teile der Anordnung antithrombotisch gerüstet, insbesondere heparinisert, sind.

## Claims

1. A cannula assembly for applying a flow cannula for introducing, circulating or diverting a flow medium in particular blood, in a human or animal body, the cannula assembly comprising:
a flow cannula (10) and a guide device (14), wherein the flow cannula (10) has a main portion (11) and a tip portion (12);
wherein the tip portion (12) is pre-bent and
in the functional position, projecting from said main portion (11) in an arc shape at an angle relative to said main portion (11); and wherein
the tip portion (12), in the application position, is maintained by said guide device (14) in a direction of a longitudinal extent of said main portion (11),
**characterized in that** the cannula assembly is configured for trans-septal cannulation of a the left atrium (2) of a heart (1) by an approach via the superior vena cava for trans-atrial puncture.

2. The cannula assembly according to claim 1, **characterized in that** the guide device (14) comprises:
- a guide wire (15, 24, 30);
- a first dilation cannula (16) to be pushed over said guide wire;
- a second dilation cannula (17) to be pushed over said first dilation cannula (10); and, optionally, one or more further dilation cannulas; and
- a guide sleeve (18) arranged over the second dilation cannula or over an outermost dilation cannula.

3. The cannula assembly according to claim 2, wherein each of said first dilation cannula, said second dilation cannula, and said optional further dilation cannulas (16; 17), if provided, has a respective tip portion (19, 20) designed, in the same way as said tip portion (12) of said flow cannula (10), pre-bent with sufficient tension and protruding elastically in an arc shape at an angle from the respective main portions (11, 25, 26), wherein said first dilation cannula (16) is guided by said guide wire (15, 24), and said second dilation cannula (17) and any optional said dilation cannula, is guided by said first dilation cannula (16) arranged therein and is guided along the longitudinal extent of said guide wire (24).

4. The cannula assembly according to any one of claims 1 to 3, **characterized in that** during application, said guide sleeve (18) is removable from said tip portion (12), to allow said tip portion to adopt the arc-shaped curvature, and that the guide sleeve (18) is removable in its entirety and replaceable by said flow cannula (10), after which said guide wire (24) and the first and the second and any further dilation cannulas (16, 17) are removable from said flow cannula (10), to free up an entire internal cross section of the flow cannula (10).

5. The cannula assembly according to any one of claims 2 to 4, **characterized in that** the tip portion (20) of said second or an outermost dilation cannula (17) has, adjacent to said tip, a dilation balloon (21) whose diameter (22) in an inflated state corresponds to an external diameter of the cannula and whose diameter (23) in a relaxed state is smaller than or equal to an internal diameter of the cannula.

6. The cannula assembly according to any one of claims 1 to 5, **characterized in that** the flow cannula (10) has a locking device (27), preferably a balloon, at said tip portion (12) for fixing the cannula (10).

7. The cannula assembly according to any one of claims 1 to 6, **characterized in that** the first, the second dilation cannula and, any optional, further dilation cannulas (16, 17), and said flow cannula (10) are bent at the tip portion (12), in an unsupported state, at an angle of 20° to 100°,
preferably at an angle of 60° to 95°,
more preferably at an angle of 90°.

8. The cannula assembly according to any one of claims 1 to 7, **characterized in that** the surfaces of components of the assembly are rendered antithrombotic, in particular heparinized.

## Revendications

1. Agencement de canule pour appliquer une canule d'écoulement pour introduire, faire passer ou dévier des milieux en écoulement, en particulier du sang, dans le corps humain ou animal, comprenant la canule d'écoulement (10) et un dispositif de guidage (14), dans lequel la canule d'écoulement (10) comprend une section principale (11) et une section de pointe (12), dans lequel la section de pointe (12) est recourbée et est conçue élastique et, en position fonctionnelle, arquée et en saillie selon un angle par rapport à la section principale (11), et dans lequel la section de pointe (12), en position d'application, est maintenue par le dispositif de guidage (14) dans la direction de l'étendue longitudinale de la section principale (11),
**caractérisé en ce que** l'agencement de canule est conçu pour la canulation transseptale de l'oreillette gauche (2) du cœur (1) avec un abord par l'intermédiaire de la veine cave supérieure pour la ponction transauriculaire.

2. Agencement de canule selon la revendication 1, **caractérisé en ce que** le dispositif de guidage (14) comporte
- un fil de guidage (15, 24, 30),
- une première canule de dilatation (16) pouvant coulisser sur le fil de guidage,
- une deuxième canule de dilatation (17) pouvant coulisser sur la première canule de dilatation (10) et éventuellement encore d'autres canules de dilatation et
- une douille de guidage (18) disposée sur la deuxième canule de dilatation ou la canule de dilatation la plus externe.

3. Agencement de canule selon la revendication 2, **caractérisé en ce que** la première et la deuxième et éventuellement chaque autre canule de dilatation (16, 17) sont recourbées avec une tension suffisante dans leurs sections de pointe (19, 20) de la même manière que la section de pointe (12) de la canule d'écoulement (10), et sont conçues élastiques, arquées et en saillie selon un angle par rapport aux sections principales respectives (11, 25, 26), dans lequel la première canule de dilatation (16) est guidée par le fil de guidage (15, 24) et la deuxième canule de dilatation (17) et éventuellement chaque autre sont guidées par la première canule de dilatation (16) y étant disposée et sont guidées le long de l'étendue longitudinale du fil de guidage (24).

4. Agencement de canule selon l'une des revendications 1 à 3, **caractérisé en ce que** la douille de guidage (18), lors de l'application, peut être retirée de la section de pointe (12) de telle sorte que celle-ci puisse adopter la courbure arquée, et **en ce que** la douille de guidage (18) peut être retirée en totalité et remplacée par la canule d'écoulement (10), après quoi le fil de guidage (24) et la première et la deuxième et toute autre canule de dilatation (16, 17) sur la canule d'écoulement (10) peuvent être retirées de telle sorte que l'ensemble de la section transversale interne de la canule d'écoulement (10) soit libérée.

5. Agencement de canule selon l'une des revendications 2 à 4, **caractérisé en ce que** la section de pointe (20) de la deuxième canule de dilatation ou canule de dilatation la plus externe (17) comprend au voisinage de la pointe un ballonnet de dilatation (21), dont le diamètre (22) à l'état gonflé est le diamètre externe de la canule et dont le diamètre (23) à l'état détendu est inférieur ou égal au diamètre interne de la canule.

6. Agencement de canule selon l'une des revendications 1 à 5, **caractérisé en ce que** la canule d'écoulement (10) comprend dans la section de pointe (12) un dispositif de blocage (27), de préférence un ballonnet, fixant la canule (10).

7. Agencement de canule selon l'une des revendications 1 à 6, **caractérisé en ce que** les première, deuxième canules de dilatation et canules de dilatation suivantes éventuelles (16, 17) et la canule d'écoulement (10) sont courbées dans la section de pointe (12), à l'état non soutenu, d'un angle de 20° à 100°, de préférence de 60° à 95°, de manière particulièrement préférée de 90°.

8. Agencement de canule selon l'une des revendications 1 à 7, **caractérisé en ce que** les surfaces des parties de l'agencement sont préparées de manière antithrombotique, en particulier héparinisées.
